# EUROPEAN PATENT APPLICATION

(11) **EP 4 523 624 A2**
(43) Date of publication of application: **19.03.2025**
(21) Application number: 25154868.1
(22) Date of filing: 14.03.2023
(51) Int. Cl.: A61B 5/154

(54) **HEMOLYSIS-REDUCTION CONNECTOR FOR DIRECT BLOOD DRAW**

(30) Priority: 01.04.2022 US 202263326706 P
(62) Divisional of application: 23714425.8
(71) Applicant: Carefusion 303 Inc., San Diego, California 92130 (US)
(72) Inventor: Salehi Borojerdi, Alireza, San Diego, 92130 (US); Jadhav, Amarsinh Deeliprao, 560045 Bengaluru (IN); Khan, Mohammed Mehtab, 560045 Bengaluru (IN); Bieritz, Shelby Ann, San Diego, 92130 (US)
(74) Representative: Zenz Patentanwälte Partnerschaft mbB

(57) **Abstract**

A flow restriction device may include a male luer connector portion and a female luer connector portion. The male luer connector portion can define a cavity and a first lumen in fluid-flow continuity with the cavity. The female luer connector portion defines a second lumen and an extension in fluid-flow continuity with the second lumen. The extension includes a first end, a second end, an outer surface, and a channel. The channel is defined on the outer surface. The channel is helically disposed around the outer surface of the extension and extends between the first and second ends. The extension is configured to be at least partially disposed within the cavity. The channel and the inner surface of the cavity define a fluid passage in fluid-flow continuity with the first lumen and the second lumen.

## Description

### TECHNICAL FIELD

The present disclosure generally relates to blood draw and administration of parenteral fluids to a patient, and particularly to systems and methods to reduce hemolysis in PIVC blood draw using a connector.

### BACKGROUND

Catheters are commonly used for a variety of infusion therapies. For example, catheters may be used for infusing fluids, such as normal saline solution, various medicaments, and total parenteral nutrition, into a patient. Catheters may also be used for withdrawing blood from the patient.

A common type of catheter is an over-the-needle peripheral intravenous ("IV") catheter (PIVC). As its name implies, the over-the-needle catheter may be mounted over an introducer needle having a sharp distal tip. A catheter assembly may include a catheter hub, the catheter extending distally from the catheter hub, and the introducer needle extending through the catheter. The catheter and the introducer needle may be assembled so that the distal tip of the introducer needle extends beyond the distal tip of the catheter with the bevel of the needle facing up away from skin of the patient. The catheter and introducer needle are generally inserted at a shallow angle through the skin into vasculature of the patient.

In order to verify proper placement of the introducer needle and/or the catheter in the blood vessel, a clinician generally confirms that there is "flashback" of blood in a flashback chamber of the catheter assembly. Once placement of the needle has been confirmed, the clinician may temporarily occlude flow in the vasculature and remove the needle, leaving the catheter in place for future blood withdrawal or fluid infusion.

For blood withdrawal or collecting a blood sample from a patient, a blood collection container may be used. The blood collection container may include a syringe. Alternatively, the blood collection container may include a test tube with a rubber stopper at one end. In some instances, the test tube has had all or a portion of air removed from the test tube so pressure within the test tube is lower than ambient pressure. Such a blood collection container is often referred to as an internal vacuum or a vacuum tube. A commonly used blood collection container is a VACUTAINER^{®} blood collection tube, available from Becton Dickinson & Company.

The blood collection container may be coupled to the catheter. When the blood collection container is coupled to the catheter, a pressure in the vein is higher than a pressure in the blood collection container, which pushes blood into the blood collection container, thus filling the blood collection container with blood. A vacuum within the blood collection container decreases as the blood collection container fills, until the pressure in the blood collection container equalizes with the pressure in the vein, and the flow of blood stops.

Unfortunately, as blood is drawn into the blood collection container, red blood cells may experience high shear stress and be susceptible to hemolysis due to the flow rate through and geometry of the blood collection system. Hemolysis may result in rejection and discard of a blood sample. The blood draw can also result in an area of local negative pressure at the catheter tip, which may lead to catheter tip collapse, vein collapse, or other complications that prevent or restrict blood from filling the blood collection container. Furthermore, blood spillage during and/or after blood draw may occur.

The description provided in the background section should not be assumed to be prior art merely because it is mentioned in or associated with the background section. The background section may include information that describes one or more aspects of the subject technology.

### SUMMARY

The present disclosure provides a flow restriction device, comprising: a male luer connector portion defining a cavity and a first lumen in fluid-flow continuity with the cavity; and a female luer connector portion defining a second lumen and an extension in fluid-flow continuity with the second lumen, the extension comprising: a first end; a second end; an outer surface; and a channel defined on the outer surface, wherein the channel is helically disposed around the outer surface of the extension and extends between the first and second ends, the extension is configured to be at least partially disposed within the cavity, and the channel and the inner surface of the cavity define a fluid passage in fluid-flow continuity with the first lumen and the second lumen.

In some instances, the present disclosure provides a flow restriction device, comprising: a male luer connector portion defining a first cavity and a first lumen in fluid-flow continuity with the first cavity; and a female luer connector portion defining a second lumen and an extension, wherein the extension defines a second cavity in fluid-flow continuity with the second lumen, the extension comprising: a first end; a second end; an outer surface; and a channel defined on the outer surface, wherein the channel is helically disposed around the outer surface of the extension and extends between the first and second ends, the extension is configured to be at least partially disposed within the first cavity, and the channel and the inner surface of the first cavity define a fluid passage in fluid-flow continuity with the first cavity and the second cavity.

In some aspects, the present disclosure provides a peripheral intravenous catheter assembly configured to limit hemolysis during drawing of blood from a patient, comprising: a catheter hub having a proximal end and a distal end; a fluid collection device; and a flow restriction device, comprising: a male luer connector portion defining a cavity and a first lumen in fluid-flow continuity with the cavity and the catheter hub; and a female luer connector portion defining a second lumen in fluid-flow continuity with the fluid collection device and an extension in fluid-flow continuity with the second lumen, the extension comprising: a first end; a second end; an outer surface; and a channel defined on the outer surface, wherein the channel is helically disposed around the outer surface of the extension and extends between the first and second ends, the extension is configured to be at least partially disposed within the cavity, and the channel and the inner surface of the cavity define a fluid passage in fluid-flow continuity with the first lumen and the second lumen.

It is understood that other configurations of the subject technology will become readily apparent to those skilled in the art from the following detailed description, wherein various configurations of the subject technology are shown and described by way of illustration. As will be realized, the subject technology is capable of other and different configurations and its several details are capable of modification in various other respects, all without departing from the scope of the subject technology. Accordingly, the drawings and detailed description are to be regarded as illustrative in nature and not as restrictive.

### BRIEF DESCRIPTION OF THE DRAWINGS

The following figures are included to illustrate certain aspects of the embodiments and should not be viewed as exclusive embodiments. The subject matter disclosed is capable of considerable modifications, alterations, combinations, and equivalents in form and function, as will occur to those skilled in the art and having the benefit of this disclosure.
FIG. 1 illustrates a vascular access device including a peripheral intravenous catheter (PIVC) assembly that includes a flow restriction device, in accordance with some embodiments of the present disclosure.
FIG. 2 illustrates a perspective view of the flow restriction device, in accordance with some embodiments of the present disclosure.
FIG. 3 illustrates an exploded view of the flow restriction device of FIG. 2, in accordance with some embodiments of the present disclosure.
FIG. 4 illustrates an exploded cross-sectional view of the flow restriction device of FIG. 2, in accordance with some embodiments of the present disclosure.

### DETAILED DESCRIPTION

The detailed description set forth below describes various configurations of the subject technology and is not intended to represent the only configurations in which the subject technology may be practiced. The detailed description includes specific details for the purpose of providing a thorough understanding of the subject technology. Accordingly, dimensions may be provided in regard to certain aspects as non-limiting examples. However, it will be apparent to those skilled in the art that the subject technology may be practiced without these specific details. In some instances, well-known structures and components are shown in block diagram form in order to avoid obscuring the concepts of the subject technology.

It is to be understood that the present disclosure includes examples of the subject technology and does not limit the scope of the appended claims. Various aspects of the subject technology will now be disclosed according to particular but non-limiting examples. Various embodiments described in the present disclosure may be carried out in different ways and variations, and in accordance with a desired application or implementation.

Blood draw via a vascular access device has drawn increasing attention attributed to minimized needle sticks and improved operation efficiency as compared with traditional blood draw methods with venipuncture. Current blood draw using a peripheral intravenous catheter (PIVC) has seen some challenges, one of the most critical is hemolysis related blood quality. In particular, with currently existing PIVC products in the market, along with the standard connection (such as a short extension set and a needleless connector), and blood collection devices (such as a Vacutainer), the shear stress exerted onto red blood cells often causes hemolysis.

Various embodiments of the present disclosure are directed to providing systems and methods to address hemolysis in PIVC blood draw with a hemolysis reduction accessory (also referred to herein as a flow restriction device), such as a connector, which is attached to the PIVC and serves as a flow restrictor to reduce risk of hemolysis. The hemolysis-reduction accessory is advantageously compatible with PIVC placement and does not necessitate change to any of the existing operations. The hemolysis-reduction accessory of the various embodiments described herein is potentially applicable to a wide variety of PIVC products, and compatible with existing blood collection devices and infusion disposables.

Various embodiments of the present disclosure focus on effective flow restriction with the add-on hemolysis-reduction accessory (also referred to herein as a flow restriction device) that regulates the overall flow rate of the entire fluid path as blood travels through. The flow restriction device can be either assembled with the PIVC or co-packaged with the PIVC. The clinician may connect a blood collection device to the port of the accessory and can then draw blood to the intended volume. After blood draw, the clinician may disconnect and discard the flow restriction device and the blood collection device together. As such, this flow restriction device can be either for single blood draw or stay inline throughout indwell.

The flow restriction devices and associated blood collection systems of the various embodiments described herein additionally provide further advantages over currently existing blood collection systems. For example, add-on flow restriction devices described herein reduce hemolysis while allowing blood to be drawn through an already-placed PIVC. Further, the flow restriction devices described herein are compatible with PIVC connections and allow for seamless blood draw at insertion. Additionally, since the flow restriction devices are an add-on which can be easily incorporated without any changes to existing PIVC, there is minimal impact to clinical setting and operations.

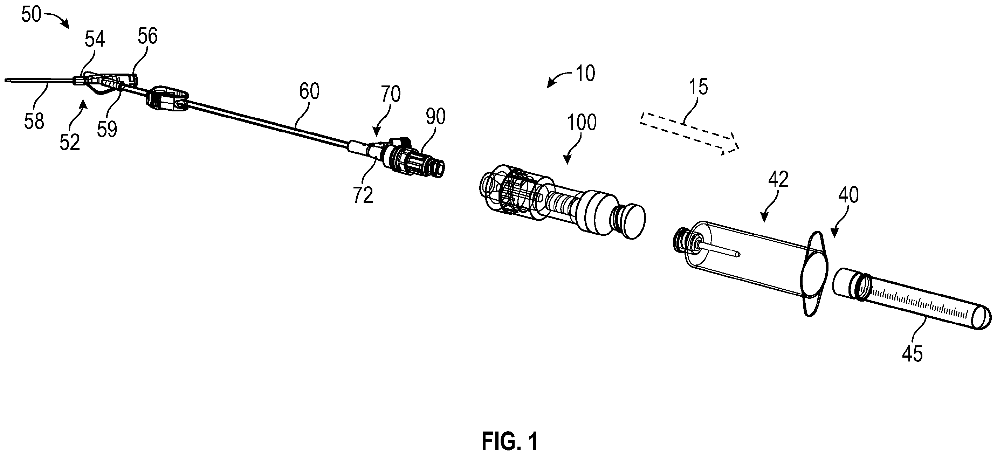

FIG. 1 illustrates a vascular access device 10 including a peripheral intravenous catheter (PIVC) assembly 50 that includes a connector or flow restriction device 100, in accordance with some embodiments of the present disclosure. The flow restriction device 100 may be configured to facilitate connections for components of the vascular access device 10 and reduce a likelihood of hemolysis during blood collection using the vascular access device 10. In some embodiments, the vascular access device 10 may include a catheter assembly 50. The catheter assembly 50 may include a catheter hub 52, which may include a distal end 54, a proximal end 56, and a lumen extending through the distal end and the proximal end. The catheter assembly 50 may further include a catheter 58, which may be secured within the catheter hub 52 and may extend distally from the distal end 54 of the catheter hub 52. In some embodiments, the catheter assembly 50 may be a peripheral intravenous catheter (PIVC).

In some embodiments, the catheter assembly 50 may include or correspond to any suitable catheter assembly 50. In some embodiments, the catheter assembly 50 may be integrated and include an extension tube 60, which may extend from and be integrated with a side port 59 of the catheter hub 52. A non-limiting example of an integrated catheter assembly is the BD NEXIVA^{™} Closed IV Catheter system, available from Becton Dickinson and Company. In some embodiments, a proximal end of the extension tube 60 may be coupled to an adapter, such as, for example, a Y-adapter 70. In some embodiments, the flow restriction device 100 may be fluidly coupled to the Y-adapter 70.

In some embodiments, the catheter assembly 50 may be non-integrated and may not include the extension tube 60. In these and other embodiments, the flow restriction device 100 may be configured to couple to the proximal end 56 of the catheter hub 52 or another suitable portion of the catheter assembly 50. In some embodiments, the flow restriction device 100 may be coupled directly to the catheter assembly 50, eliminating the extension tube 60 and providing a compact catheter system.

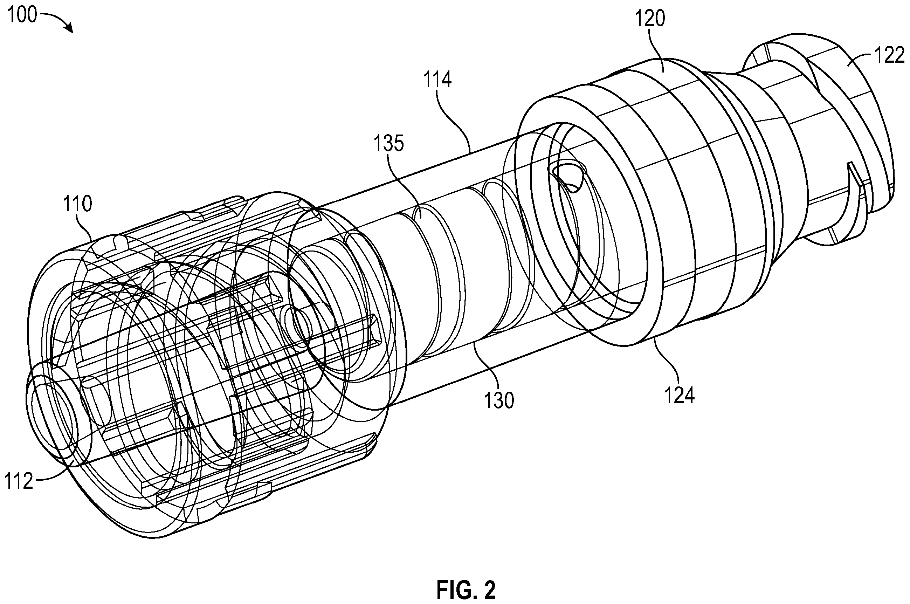

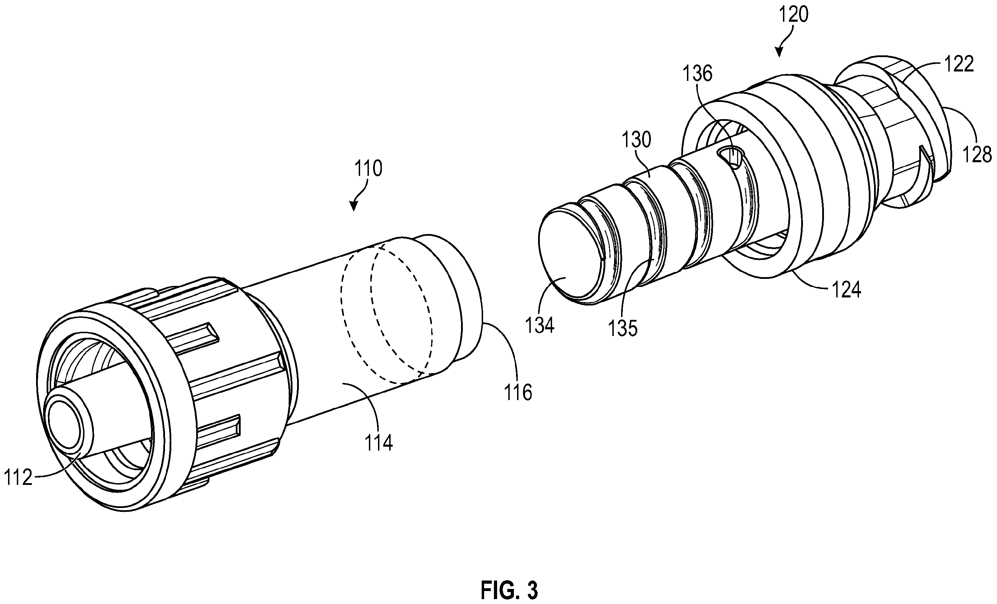

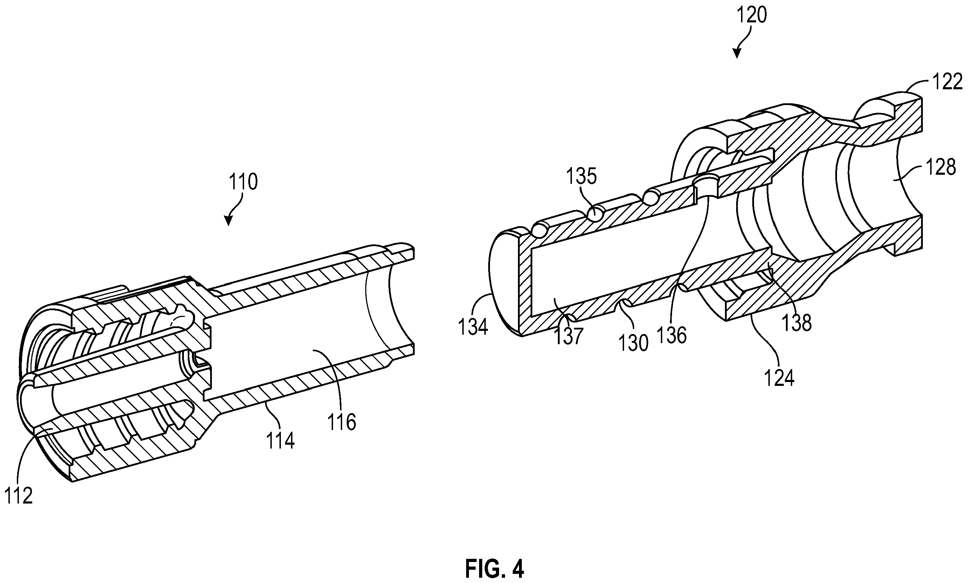

FIG. 2 illustrates a perspective view of the flow restriction device 100, in accordance with some embodiments of the present disclosure. FIG. 3 illustrates an exploded view of the flow restriction device 100 of FIG. 2, in accordance with some embodiments of the present disclosure. FIG. 4 illustrates an exploded cross-sectional view of the flow restriction device 100 of FIG. 2, in accordance with some embodiments of the present disclosure.

As illustrated in FIGS. 2-4, and with continued reference to FIG. 1, in some embodiments, the flow restriction device 100 may include a male luer connector portion 110 configured to couple to the catheter assembly 50. The male luer connector portion 110 may have an internal surface defining a lumen 112 of the male luer connector portion 110. In some embodiments, a distal end of the male luer connector portion 110 can be coupled to the catheter assembly 50. In some embodiments, the lumen 112 is in fluid-flow continuity with a cavity 116 defined by the body 114 of the male luer connector portion 110.

In some embodiments, the flow restriction device 100 may further include a female luer connector portion 120 disposed proximally to the male luer connector portion 110. The female luer connector portion 120 may be configured to couple to fluid collection device 40 (e.g., a blood collection device). For example, the female luer connector portion 120 may be integrated with the blood collection device 40 or monolithically formed with the blood collection device 40 as a single unit or piece. As another example, the female luer connector portion 120 may be in the form of a female luer connector or another suitable connector, which may be coupled with a male luer portion of the blood collection device 40. The female luer connector portion 120 may include an internal surface defining a lumen 128 extending therethrough for coupling to the male luer portion of the blood collection device 40. In some embodiments, a proximal end of the female luer connector portion 120 can be coupled to the blood collection device 40. Optionally, a threaded portion 122 of the female luer connector portion 120 can be utilized to engage the female luer connector portion 120 to a corresponding male luer portion.

In the depicted example, the body 114 of the male luer connector portion 110 can be coupled to the body 124 of the female luer connector portion 120 to allow fluid-flow continuity between the lumen 112 and the lumen 128 of the flow restriction device 100 and form a common housing.

In the depicted example, the female luer connector portion 120 can include an extension 130 to control fluid flow between the lumens 112, 128 and reduce hemolysis through the total blood flow pathway from PIVC to blood collection device 100. As illustrated, the extension 130 can be disposed within the cavity 116 formed by the male luer connector portion 110. As illustrated, the extension 130 directs fluid flow between the lumens 112 and 128 of the male luer connector portion 110 and the female luer connector portion 120, respectively while inducing a resistance to the fluid flow 130. By inducing a resistance to the fluid flow, the rate of fluid flow through the assembly from PIVC to blood collection device is reduced 130. Reducing the flow rate of a fluid, such as blood, through the assembly can reduce the hemolysis index of the blood.

In some embodiments, an extension 130 defines a groove or channel 135 that directs fluid flow from a distal end 134 of the extension 130 to a proximal end 138 of the extension 130. As illustrated, the channel 135 can be defined along an outer surface of the extension 130. Accordingly, when the extension 130 is positioned within the cavity 116 of the male luer connector portion 110, at least a portion of the outer surface of the extension 130 can engage against an inner surface of the cavity 116 to permit the channel 135 and the inner surface of the cavity 116 to cooperatively define a fluid passage therebetween. The defined fluid passage can extend along a path between the distal end 134 and the proximal end 138 of the extension 130 to reduce a rate of fluid flow across the extension 130. In some embodiments, the groove or channel 135 can be formed on an inner surface of the body 114 such that engagement with the outer surface of the extension 130 forms a fluid pathway. In some embodiments, the groove or channel 135 can extend along one or both of the inner surface of the body 114 and the outer surface of the extension 130 such that when the body 114 receives the extension 130, a fluid pathway is formed to conduct fluid between the body 114 and the extension 130.

In the depicted example, fluid flow can pass from the lumen 112 of the male luer connector portion 110 to the distal end 134 of the extension 130 via the cavity 116. In some embodiments, fluid can flow from the proximal end 138 of the extension 130 to the lumen 128 of the female luer connector portion 120 via a port 136 formed through the extension 130. In some embodiments, the port 136 can allow fluid flow between the proximal end 138 of the extension 130 and/or the channel 135 into a cavity 137 formed within the extension 130. The cavity 137 formed within the extension 130 can be separated from the cavity 116 of the male luer connector portion 110 by the body of the extension 130. As illustrated, the cavity 137 can be in fluid-flow continuity with the lumen 128. Therefore, in some embodiments, fluid flow can pass from the proximal end 138 of the extension 130 to the lumen 128 via a port 136 and the cavity 137.

Therefore, in some embodiments, the channel 135 (in conjunction with the inner surface of the cavity 116) can direct fluid flow from the male luer connector portion 110 into the fluid collection device 40 via the female luer connector portion 120. As depicted, the channel 135 may provide a fluid pathway from the catheter assembly 50 to the fluid collection device 40 via the flow restriction device 100. For example, in some embodiments, a leg 72 of the Y-adapter 70 may be coupled to the flow restriction device 100. The leg 72 of Y-adapter 70 may include a lumen into which the distal end of the male luer connector portion 110 may be coupled. The Y-adapter 70 may provide fluid-flow continuity between the flow restriction device 100 via a connector 90, which is depicted as a needleless connector, and the channel 135 with the catheter assembly 50, for example, via the extension tubing 60. Accordingly, the channel 135 may define a fluid pathway with a helical, spiral, extended, or otherwise indirect path (as discussed below) through which fluid entering the flow restriction device 100 from the catheter assembly 50, may flow through the flow restriction device 100 for collection in the fluid collection device 40. For example, where blood is being withdrawn or collected from a patient, the medical fluid may be blood, and the fluid collection device 40 may be a blood collection device. In some embodiments, the blood collection device may be a blood collection tube holder such as the BD Vacutainer ^{®} LuerLok^{™} Access Device (LLAD). Accordingly, during blood collection or withdrawal from the patients, the blood sample may flow from the distal end of the male luer connector portion 110 into the LLAD 40 via the flowpath or channel 135.

In some embodiments, the extension 130 can define a longitudinal axis that extends through the distal and proximal ends 134, 138. The channel 135 spans between the distal and proximal ends 134, 138 of the extension 130, and is configured to spiral or helically extend along the longitudinal axis. Because the path spirals about the longitudinal axis, the channel 135 extends indirectly between the distal and proximal ends 134, 138 of the extension 130. The indirect path between the distal and proximal ends 134, 138 of the extension 130 can have a length that is greater than a distance between the distal and proximal ends 134, 138 of the extension 130. In some instances, the pitch and the angle of the turns of the helical channel 135 can be optimized to reduce the volumetric flow rate of a fluid, e.g., blood, moving through the channel 135 by inducing resistance to the fluid. Since the decreased blood flow rate results in a reduction in shear stress experienced by the red blood cells, the risk of hemolysis during blood collection may advantageously be reduced.

The flow restriction device 100 of the various embodiments described herein is advantageous over currently existing PIVC blood collection systems. For example, during blood draws with currently existing PIVCs, blood cells may experience supraphysiologic levels of shear stress as they flow from the distal end to the proximal end of the blood collection systems. Subjecting red blood cells to supraphysiologic levels of shear stress is considered a major source of mechanical damage and hemolysis of red blood cells The diameter and effective length of the channel 135 may facilitate increased flow resistance within the vascular access system to reduce the flow rate and reduce shear stress experienced by the red blood cells 15. For example, the minimized diameter and elongated effective length of the channel 135 may provide increased resistance to flow of the blood 15 and thereby decrease blood flow rate within the PIVC, device, and blood collection device assembly 100. Since the decreased blood flow rate causes a reduction in shear stress experienced by the red blood cells 15, the risk of hemolysis during blood collection may advantageously be reduced. Further, the geometry of the channel 135 can reduce the amount of blood wasted by currently existing PIVC blood collection systems.

Further, the flow restriction device 100 can reduce hemolysis without the use of active devices such as valves or other flow control devices. In some applications, the configuration of the flow restriction device 100 as described herein can minimize flushing of the vascular access device 10, minimize dead volume, and facilitate a compact design. Further, the configuration of the flow restriction device 100 can facilitate ease of manufacturing compared to certain prior art designs. For example, in some applications, the male luer connector portion 110 and the female luer connector portion 120 can be joined together followed by ultrasonic welding to form the flow restriction device 100.

### Illustration of Subject Technology as Clauses

The subject technology is illustrated, for example, according to various aspects described below. Various examples of aspects of the subject technology are described as numbered clauses (1, 2, 3, etc.) for convenience. These are provided as examples and do not limit the subject technology. It is noted that any of the dependent clauses may be combined in any combination, and placed into a respective independent clause, e.g., clause 1 or clause 5. The other clauses can be presented in a similar manner.

Clause 1. A flow restriction device, comprising: a male luer connector portion defining a cavity and a first lumen in fluid-flow continuity with the cavity; and a female luer connector portion defining a second lumen and an extension in in fluid-flow continuity with the second lumen, the extension comprising: a first end; a second end; an outer surface; and a channel defined on the outer surface, wherein the channel is helically disposed around the outer surface of the extension and extends between the first and second ends, the extension is configured to be at least partially disposed within the cavity, and the channel and the inner surface of the cavity define a fluid passage in fluid-flow continuity with the first lumen and the second lumen.

Clause 2. The flow restriction device of Clause 1, wherein the extension defines a second cavity disposed within the extension, and the second lumen is in fluid-flow continuity with the second cavity.

Clause 3. The flow restriction device of Clause 1, wherein the channel is in fluid-flow continuity with the second lumen via a port extending through the extension.

Clause 4. The flow restriction device of Clause 1, wherein the fluid passage is configured to increase flow resistance through the fluid passage to reduce shear stress experienced by the fluid.

Clause 5. The flow restriction device of Clause 1, wherein the first lumen is configured to be coupled to a catheter hub.

Clause 6. The flow restriction device of Clause 1, wherein the second lumen is configured to couple to a fluid collection device.

Clause 7. A flow restriction device, comprising: a male luer connector portion defining a first cavity and a first lumen in fluid-flow continuity with the first cavity; and a female luer connector portion defining a second lumen and an extension, wherein the extension defines a second cavity in fluid-flow continuity with the second lumen, the extension comprising: a first end; a second end; an outer surface; and a channel defined on the outer surface, wherein the channel is helically disposed around the outer surface of the extension and extends between the first and second ends, the extension is configured to be at least partially disposed within the first cavity, and the channel and the inner surface of the first cavity define a fluid passage in fluid-flow continuity with the first cavity and the second cavity.

Clause 8. The flow restriction device of Clause 7, wherein the channel is in fluid-flow continuity with the second cavity via a port extending through the extension.

Clause 9. The flow restriction device of Clause 7, wherein the fluid passage is configured to increase flow resistance through the fluid passage to reduce shear stress experienced by the fluid. Clause 10. The flow restriction device of Clause 7, wherein the first lumen is configured to be coupled to a catheter hub.

Clause 11. The flow restriction device of Clause 7, wherein the second lumen is configured to be coupled to a fluid collection device.

Clause 12. A peripheral intravenous catheter assembly configured to limit hemolysis during drawing of blood from a patient, comprising: a catheter hub having a proximal end and a distal end; a fluid collection device; and a flow restriction device, comprising: a male luer connector portion defining a cavity and a first lumen in fluid-flow continuity with the cavity and the catheter hub; and a female luer connector portion defining a second lumen in fluid-flow continuity with the fluid collection device and an extension in fluid-flow continuity with the second lumen, the extension comprising: a first end; a second end; an outer surface; and a channel defined on the outer surface, wherein the channel is helically disposed around the outer surface of the extension and extends between the first and second ends, the extension is configured to be at least partially disposed within the cavity, and the channel and the inner surface of the cavity define a fluid passage in fluid-flow continuity fluid-flow continuity with the first lumen and the second lumen.

Clause 13. The peripheral intravenous catheter assembly of Clause 12, wherein the extension defines a second cavity disposed within the extension, and the second lumen is in fluid-flow continuity fluid-flow continuity with the second cavity.

Clause 14. The peripheral intravenous catheter assembly of Clause 12, wherein the channel is in fluid-flow continuity with the second lumen via a port extending through the extension.

Clause 15. The peripheral intravenous catheter assembly of Clause 12, wherein the fluid passage is configured to increase flow resistance through the fluid passage to reduce shear stress experienced by the fluid.

Clause 16. The peripheral intravenous catheter assembly of Clause 12, wherein the first lumen is configured to be coupled to a catheter hub.

Clause 17. The peripheral intravenous catheter assembly of Clause 12, wherein the second lumen is configured to couple to a fluid collection device.

The present disclosure is provided to enable any person skilled in the art to practice the various aspects described herein. The disclosure provides various examples of the subject technology, and the subject technology is not limited to these examples. Various modifications to these aspects will be readily apparent to those skilled in the art, and the generic principles defined herein may be applied to other aspects.

A reference to an element in the singular is not intended to mean "one and only one" unless specifically so stated, but rather "one or more." Unless specifically stated otherwise, the term "some" refers to one or more. Pronouns in the masculine (e.g., his) include the feminine and neuter gender (e.g., her and its) and vice versa. Headings and subheadings, if any, are used for convenience only and do not limit the invention.

The word "exemplary" is used herein to mean "serving as an example or illustration." Any aspect or design described herein as "exemplary" is not necessarily to be construed as preferred or advantageous over other aspects or designs. In one aspect, various alternative configurations and operations described herein may be considered to be at least equivalent.

As used herein, the phrase "at least one of" preceding a series of items, with the term "or" to separate any of the items, modifies the list as a whole, rather than each item of the list. The phrase "at least one of" does not require selection of at least one item; rather, the phrase allows a meaning that includes at least one of any one of the items, and/or at least one of any combination of the items, and/or at least one of each of the items. By way of example, the phrase "at least one of A, B, or C" may refer to: only A, only B, or only C; or any combination of A, B, and C.

A phrase such as an "aspect" does not imply that such aspect is essential to the subject technology or that such aspect applies to all configurations of the subject technology. A disclosure relating to an aspect may apply to all configurations, or one or more configurations. An aspect may provide one or more examples. A phrase such as an aspect may refer to one or more aspects and vice versa. A phrase such as an "embodiment" does not imply that such embodiment is essential to the subject technology or that such embodiment applies to all configurations of the subject technology. A disclosure relating to an embodiment may apply to all embodiments, or one or more embodiments. An embodiment may provide one or more examples. A phrase such an embodiment may refer to one or more embodiments and vice versa. A phrase such as a "configuration" does not imply that such configuration is essential to the subject technology or that such configuration applies to all configurations of the subject technology. A disclosure relating to a configuration may apply to all configurations, or one or more configurations. A configuration may provide one or more examples. A phrase such a configuration may refer to one or more configurations and vice versa. In one aspect, unless otherwise stated, all measurements, values, ratings, positions, magnitudes, sizes, and other specifications that are set forth in this specification, including in the claims that follow, are approximate, not exact. In one aspect, they are intended to have a reasonable range that is consistent with the functions to which they relate and with what is customary in the art to which they pertain.

It is understood that the specific order or hierarchy of steps, or operations in the processes or methods disclosed are illustrations of exemplary approaches. Based upon implementation preferences or scenarios, it is understood that the specific order or hierarchy of steps, operations or processes may be rearranged. Some of the steps, operations or processes may be performed simultaneously. In some implementation preferences or scenarios, certain operations may or may not be performed. Some or all of the steps, operations, or processes may be performed automatically, without the intervention of a user. The accompanying method claims present elements of the various steps, operations or processes in a sample order, and are not meant to be limited to the specific order or hierarchy presented.

All structural and functional equivalents to the elements of the various aspects described throughout this disclosure that are known or later come to be known to those of ordinary skill in the art are expressly incorporated herein by reference and are intended to be encompassed by the claims.

The following clauses correspond to the content of the claims of the parent application and serve to incorporate their disclosure into this application. These clauses are intended to preserve and carry over the original disclosure, without introducing any additional subject matter or limitations beyond the original scope.

Clause 1: A flow restriction device, comprising a male luer connector portion defining a cavity and a first lumen in fluid-flow continuity with the cavity; and a female luer connector portion defining a second lumen and an extension in fluid-flow continuity with the second lumen, the extension comprising a first end, a second end, an outer surface, and a channel defined on the outer surface, wherein the channel is helically disposed around the outer surface of the extension and extends between the first and second ends, the extension is configured to be at least partially disposed within the cavity, and the channel and the inner surface of the cavity define a fluid passage in fluid-flow continuity with the first lumen and the second lumen.

Clause 2: The flow restriction device of Clause 1, wherein the extension defines a second cavity disposed within the extension, and the second lumen is in fluid-flow continuity with the second cavity.

Clause 3: The flow restriction device of Clause 1, wherein the channel is in fluid-flow continuity with the second lumen via a port extending through the extension.

Clause 4: The flow restriction device of Clause 1, wherein the fluid passage is configured to increase flow resistance through the fluid passage to reduce shear stress experienced by the fluid. Clause 5: The flow restriction device of Clause 1, wherein the first lumen is configured to be coupled to a catheter hub.

Clause 6: The flow restriction device of Clause 1, wherein the second lumen is configured to be coupled to a fluid collection device.

Clause 7: A flow restriction device, comprising a male luer connector portion defining a first cavity and a first lumen in fluid-flow continuity with the first cavity; and a female luer connector portion defining a second lumen and an extension, wherein the extension defines a second cavity in fluid-flow continuity with the second lumen, the extension comprising a first end, a second end, an outer surface, and a channel defined on the outer surface, wherein the channel is helically disposed around the outer surface of the extension and extends between the first and second ends, the extension is configured to be at least partially disposed within the first cavity, and the channel and the inner surface of the first cavity define a fluid passage in fluid-flow continuity with the first cavity and the second cavity.

Clause 8: The flow restriction device of Clause 7, wherein the channel is in fluid-flow continuity with the second cavity via a port extending through the extension.

Clause 9: The flow restriction device of Clause 7, wherein the fluid passage is configured to increase flow resistance through the fluid passage to reduce shear stress experienced by the fluid. Clause 10: The flow restriction device of Clause 7, wherein the first lumen is configured to be coupled to a catheter hub.

Clause 11: The flow restriction device of Clause 7, wherein the second lumen is configured to be coupled to a fluid collection device.

Clause 12: A peripheral intravenous catheter assembly configured to limit hemolysis during drawing of blood from a patient, comprising a catheter hub having a proximal end and a distal end, a fluid collection device, and a flow restriction device, comprising a male luer connector portion defining a cavity and a first lumen in fluid-flow continuity with the cavity and the catheter hub; and a female luer connector portion defining a second lumen in fluid-flow continuity with the fluid collection device and an extension in fluid-flow continuity with the second lumen, the extension comprising a first end, a second end, an outer surface, and a channel defined on the outer surface, wherein the channel is helically disposed around the outer surface of the extension and extends between the first and second ends, the extension is configured to be at least partially disposed within the cavity, and the channel and the inner surface of the cavity define a fluid passage in fluid-flow continuity with the first lumen and the second lumen.

Clause 13: The peripheral intravenous catheter assembly of Clause 12, wherein the extension defines a second cavity disposed within the extension, and the second lumen is in fluid-flow continuity with the second cavity.

Clause 14: The peripheral intravenous catheter assembly of Clause 12, wherein the channel is in fluid-flow continuity with the second lumen via a port extending through the extension.

Clause 15: The peripheral intravenous catheter assembly of Clause 12, wherein the fluid passage is configured to increase flow resistance through the fluid passage to reduce shear stress experienced by the fluid.

Clause 16: The peripheral intravenous catheter assembly of Clause 12, wherein the first lumen is configured to be coupled to a catheter hub.

Clause 17: The peripheral intravenous catheter assembly of Clause 12, wherein the second lumen is configured to be coupled to a fluid collection device.

## Claims

1. A flow restriction device, comprising:
a male luer connector portion defining a cavity and a first lumen in fluid-flow continuity with the cavity; and
a female luer connector portion defining a second lumen and an extension in fluid-flow continuity with the second lumen, wherein
an end of the cavity of the male luer connector portion is configured to couple to the female luer connector portion within a body of the female luer connector portion,
the extension is configured to be at least partially disposed within the cavity, and the extension comprises a channel defining a fluid passage in fluid-flow continuity with the first lumen and the second lumen.

2. The flow restriction device of Claim 1, wherein the extension defines a second cavity disposed within the extension, and the second lumen is in fluid-flow continuity with the second cavity.

3. The flow restriction device of Claim 1, wherein the channel is in fluid-flow continuity with the second lumen via a port extending through the extension.

4. The flow restriction device of Claim 1, wherein the fluid passage is configured to increase flow resistance through the fluid passage.

5. The flow restriction device of Claim 1, wherein the first lumen is configured to be coupled to a catheter hub.

6. The flow restriction device of Claim 1, wherein the second lumen is configured to coupled to a fluid collection device.

7. A peripheral intravenous catheter assembly configured to limit hemolysis during drawing of blood from a patient, comprising:
a catheter hub having a proximal end and a distal end;
a fluid collection device; and
a flow restriction device, comprising:
a male luer connector portion defining a cavity and a first lumen in fluid-flow continuity with the cavity and the catheter hub; and
a female luer connector portion defining a second lumen in fluid-flow continuity with the fluid collection device and an extension in fluid-flow continuity with the second lumen, wherein
an end of the cavity of the male luer connector portion is configured to couple to the female luer connector portion within a body of the female luer connector portion,
the extension is configured to be at least partially disposed within the cavity, and
the extension comprises a channel defining a fluid passage in fluid-flow continuity with the first lumen and the second lumen.

8. The peripheral intravenous catheter assembly of Claim 7, wherein the extension defines a second cavity disposed within the extension, and the second lumen is in fluid-flow continuity with the second cavity.

9. The peripheral intravenous catheter assembly of Claim 7, wherein the channel is in fluid-flow continuity with the second lumen via a port extending through the extension.

10. The peripheral intravenous catheter assembly of Claim 7, wherein the fluid passage is configured to increase flow resistance through the fluid passage.

11. The peripheral intravenous catheter assembly of Claim 7, wherein the first lumen is configured to be coupled to a catheter hub.

12. The peripheral intravenous catheter assembly of Claim 7, wherein the second lumen is configured to coupled to the fluid collection device.
